(19) Europäisches Patentamt
European Patent Office
Office européen des brevets

(11) EP 4 600 641 A1

(12) **EUROPEAN PATENT APPLICATION**
published in accordance with Art. 153(4) EPC

(43) Date of publication:
**13.08.2025 Bulletin 2025/33**

(21) Application number: **23874908.9**

(22) Date of filing: **04.10.2023**

(51) International Patent Classification (IPC):
***G01N 31/00*** *(2006.01)* ***A61K 47/16*** *(2006.01)*
***G01N 33/15*** *(2006.01)*

(52) Cooperative Patent Classification (CPC):
**A61K 47/16; G01N 31/00; G01N 33/15**

(86) International application number:
**PCT/JP2023/036245**

(87) International publication number:
**WO 2024/075783 (11.04.2024 Gazette 2024/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.10.2022 US 202263413327 P**
**13.01.2023 US 202363438897 P**

(71) Applicant: **Sawai Pharmaceutical Co., Ltd.**
**Yodogawa-ku**
**Osaka-shi**
**Osaka 532-0003 (JP)**

(72) Inventors:
- **YAMAMOTO Hiroyuki**
  **Osaka City, Osaka 532-0003 (JP)**
- **KAGAWA Chino**
  **Osaka City, Osaka 532-0003 (JP)**
- **KATSUMOTO Takaki**
  **Osaka City, Osaka 532-0003 (JP)**
- **NAKAGOMI Jun**
  **Osaka City, Osaka 532-0003 (JP)**
- **MIURA Masaru**
  **Osaka City, Osaka 532-0003 (JP)**

(74) Representative: **Stolmár & Partner**
**Patentanwälte PartG mbB**
**Blumenstraße 17**
**80331 München (DE)**

# (54) METHOD FOR QUANTIFYING REACTIVE NOX, AND FORMULATION FOR SUPPRESSING GENERATION OF NITROSO COMPOUNDS

(57) An embodiment of the present invention provides a method for quantifying reactive NOx which is a source of NOx that may be involved in the generation of nitroso compounds. Alternatively, an embodiment of the present invention provides a formulation that suppresses the generation of a nitroso compound, designed based on a method for quantifying reactive NOx that may be involved in the generation of the nitroso compounds. In one embodiment, a method for quantifying reactive NOx includes adding an amine source to a sample to generate a nitroso compound. In one embodiment, nitroso compounds may be generated by heating under a sealed condition.

EP 4 600 641 A1

FIG. 1

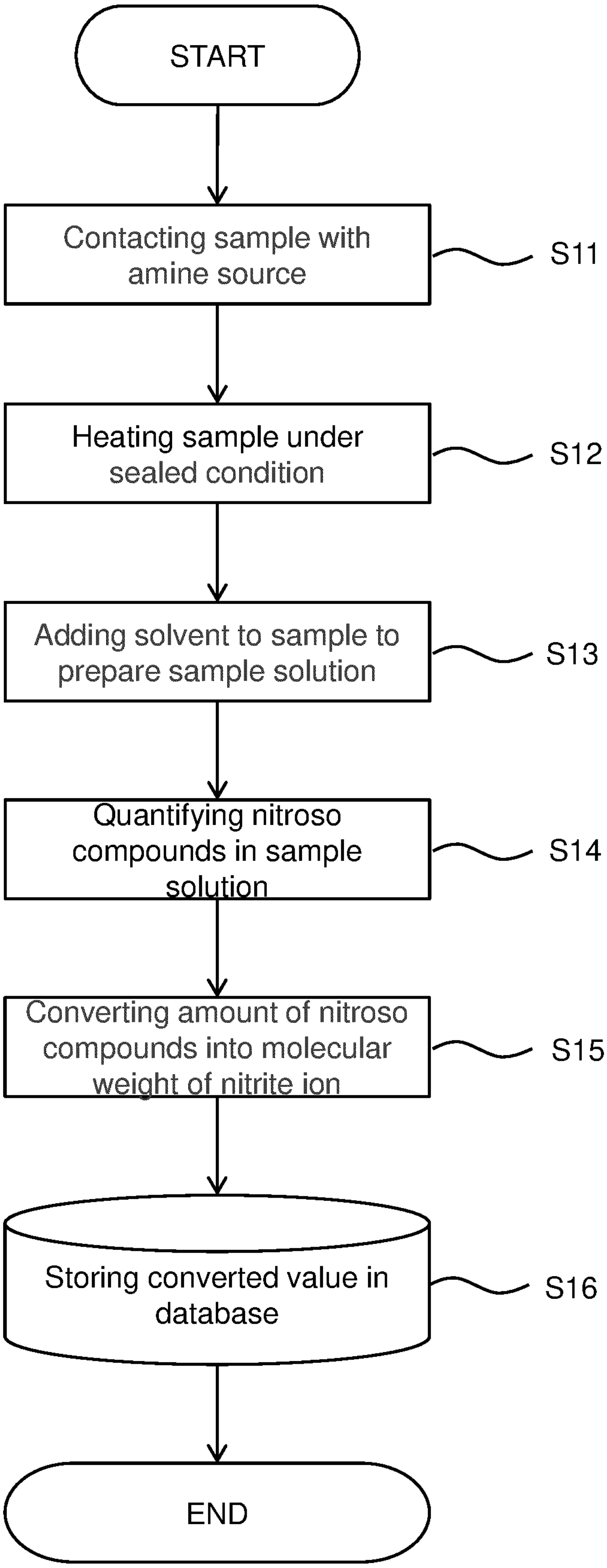
START
Contacting sample with amine source
S11
Heating sample under sealed condition
S12
Adding solvent to sample to prepare sample solution
S13
Quantifying nitroso compounds in sample solution
S14
Converting amount of nitroso compounds into molecular weight of nitrite ion
S15
Storing converted value in database
S16
END

## Description

### TECHNICAL FIELD

An embodiment of the present invention relates to a method for quantifying reactive NOx, and in particular to a method for quantifying reactive NOx contained in an active pharmaceutical ingredient, an additive agent, or a formulation for pharmaceutical use. Alternatively, an embodiment of the present invention relates to a formulation designed based on the method for quantifying reactive NOx, in which generation of nitroso compounds is suppressed.

### BACKGROUND ART

Nitroso compounds are organic compounds with nitroso groups, and nitrosamine is a type of nitroso compound generated by nitrosation of amines (substitution of the hydrogen on the amine nitrogen with a nitroso group) with an NOx source such as nitrous acid. Since some nitroso compounds have been pointed out to be carcinogenic risks, the importance of evaluating nitroso compounds in pharmaceutical preparations has been suggested in recent years. In pharmaceutical preparations, the active pharmaceutical ingredient may be an amine or an amide, which may react with NOx sources contained in the formulation to increase the amount of nitroso compounds during storage. From this, the generation of nitroso compounds can be suppressed by removing either the amine source or NOx source from the formulation. However, if the active pharmaceutical ingredient itself is an amine or amide, it is impossible to remove an amine source. Further, NOx sources are also known to contaminate active pharmaceutical ingredients and additive agents in trace amounts (for example, Non Patent Literature 1), and methods are known for measuring the content of nitrite in additive agents. Various other compounds are also contained in additive agents as NOx sources, but not all of them are considered to contribute to the formation reaction of nitroso compounds. Therefore, it is not practical to identify and control all individual NOx sources.

### CITATION LIST

### NON PATENT LITERATURE

Non Patent Literature 1: R. Boetzel et al. A Nitrite Excipient Database: A Useful Tool to Support N-Nitrosamine Risk Assessments for Drug Products. J. Pharm. Sci.2023;112: 1615-1624.

### SUMMARY OF INVENTION

### TECHNICAL PROBLEM

One object of the present invention is to provide a method for quantifying reactive Nox which is an NOx source that may be involved in the generation of nitroso compounds.

Alternatively, one object of the present invention is to provide a formulation that is designed based on a method for the quantifying reactive NOx, which may be involved in the generation of nitroso compounds, and that suppresses the generation of nitroso compounds.

### SOLUTION TO PROBLEM

According to one embodiment of the invention, there is provided a method for quantifying reactive NOx, including contacting a sample with an amine source to generate a nitroso compound.

The sample contacted with the amine source under a sealed condition may be heated to generate the nitroso compound.

The nitroso compound may be quantified, and converted by a molecular weight of nitrite ion.

The amine source may be a secondary amine, a tertiary amine, or a quaternary amine.

The sample may be one selected from a group consisting of an active pharmaceutical ingredient, a pharmaceutically acceptable additive, a pharmaceutical composition, and a formulation.

According to one embodiment of the invention, there is provided a formulation containing an active pharmaceutical ingredient or a pharmaceutically acceptable additive that has been evaluated by any of the above methods for quantifying reactive NOx.

The formulation may contain the active pharmaceutical ingredient or the pharmaceutically acceptable additive agent in which a theoretical value of nitroso compounds generated in the formulation is calculated as an amount of substance per tablet by using a total amount of reactive NOx per tablet and the theoretical value is below an acceptable

limit value of the nitroso compound calculated as the amount of substance per tablet.

## ADVANTAGEOUS EFFECTS OF INVENTION

According to one embodiment of the present invention, there is provided a method for quantifying reactive Nox which is an NOx source that may be involved in the generation of nitroso compound. Alternatively, according to one embodiment of the present invention, there is provided a formulation designed based on the method for quantifying reactive NOx, which may be involved in the generation of the nitroso compound, in which the generation of the nitroso compound is suppressed.

## BRIEF DESCRIPTION OF THE DRAWING

FIG. 1 is a flow diagram showing a method for quantifying reactive Nox according to one embodiment of the present application.
FIG. 2 is a table showing formulations of Examples and Comparative examples.
FIG. 3 shows changes in an amount of nitroso compounds when the formulations of Comparative example 1 and Example 2 were stored at 25°C and 60% RH.
FIG. 4 shows changes in an amount of nitroso compounds when the formulations of the Comparative example 1 and the Example 2 were stored at 40°C and 75% RH.
FIG. 5 shows changes in an amount of nitroso compounds when the formulations of Comparative example 2 and Example 3 were stored at 25°C and 60% RH.
FIG. 6 shows changes in an amount of nitroso compounds when the formulations of the Comparative example 2 and the Example 3 were stored at 40°C and 75% RH.

## DESCRIPTION OF EMBODIMENTS

A method for quantifying reactive NOx and a formulation in which the generation of nitroso compounds is suppressed are described below with reference to the drawings. In addition, the method for quantifying reactive NOx and the formulation in which the generation of nitroso compounds is suppressed is not to be interpreted as limited to the contents described in the following Embodiments and Examples. In addition, in the drawings referred to in the embodiment and the examples described below, identical parts or parts having similar functions are marked with the same symbol, and repeated descriptions thereof are omitted.

In this description, "nitroso compound" is a generic term for compounds having a nitroso group represented by a Chemical formula 1 below. In other words, "nitroso compound" is a generic term for compounds having a structure in which a nitroso group is attached to the nitrogen of amine, amide and the like. As a nitroso compound, although N-nitrosonornicotine (NNN), 4-(methylnitrosamino)-1-(3-pyridyl)-1-butanone (NNK), 4-(methylnitrosamino)-1-(3-pyridyl)-1-butanol (NNAL), N,N'-dinitrosopentamethylenetetramine (DPT), N-nitrosomethylethylamine (NMEA), N- nitrosopiperidine (NPIP), N-nitrosopyrrolidine (NPYR), N-nitrosomorpholine (NMOR), N-nitrosoproline (NPRO), N-nitrososarcosine (NSAR), N-nitrosodimethylamine (NDMA), N-nitrosodiethylamine (NDEA), N nitrosomethylphenylamine (NMPA), N-nitrosodiisopropylamine (NDIPA), N-nitrosoethylisopropylamine (NEIPA), N N-nitrosodibutylamine (NDBA), and N-nitrosomethylaminobutyric acid (NMBA) can be use as examples, nitroso compounds are not limited to these.

[Chemical Formula 1]

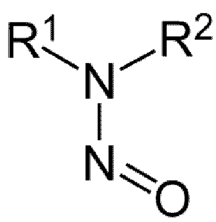

In particular, NDMA, NDEA, NMPA, NDIPA, NEIPA, NDBA, and NMBA are nitroso compounds that may be present as impurities in active pharmaceutical ingredients and pharmaceutical preparations listed in Control of Nitrosamine Impurities in Human Drugs, Guidance for Industry, February 2021, Revision 1.

In this specification, "NOx source" is a generic term for compounds containing nitroso groups capable of

generating nitroso compounds by a reaction with an amine source. In addition, NOx is synonymous with nitrogen oxide. As NOx sources, in addition to nitrite, nitric ion and nitrite salts, although nitric acid and nitrates, Nitrous acidium ion, nitrite esters, peroxynitrite, nitrosonium ion, nitro compounds, nitric anhydride, dinitrogen tetroxide, nitrosyl halides, nitrosyl thiocyanate, nitrosophenols, nitrosothiols, aqua regia, nitric oxide, nitrogen dioxide, and nitrile chloride can be use as examples, the NOx sources are not limited to those.

In this description, "reactive NOx" refers to NOx that contributes to the generation of nitroso compounds by a reaction with an amine source among the NOx sources in a sample to be measured. Since the reactivity of NOx sources to amine sources differs depending on the NOx source, NOx that contributes to the generation of nitroso compounds in the sample to be measured is quantified as reactive NOx.

In this specification, "amine source" is a generic term for amine compounds capable of generating nitroso compounds by a reaction with NOx sources. Further, amine sources also include compounds that nitrosate, such as urea and amide compounds. In an embodiment, secondary, tertiary, or quaternary amines can be used as amine sources. Since reactivity to the NOx source differs depending on the amine source, it is preferable to use an amine source that contributes to the generation of the nitroso compound to be measured. In addition, in one embodiment, from the viewpoint of suppressing the generation of nitroso compounds in pharmaceutical compositions or formulations, more accurate quantifying of reactive NOx can be achieved if an active pharmaceutical ingredient or pharmaceutically acceptable additive to be added to them is used as an amine source.

In this specification, "sample" is an additive agent, an active pharmaceutical ingredient, or a pharmaceutical composition containing reactive NOx.

In this specification, "acceptable limit value" is an acceptable content of a nitroso compound in a pharmaceutical composition or formulation, and is a daily intake of a mutagenic impurity equivalent to a theoretical carcinogenic risk of 1 in 100,000. The acceptable limit values are specified by notifications issued by regulatory authorities (for example, voluntary inspection on the risk of nitrosamine contamination in pharmaceutical preparations dated October 8, 2021 and, calculation of limit values based on structure-activity relationships by the Recommended Acceptable Intake Limits for Nitrosamine Drug Substance (NDSRIs) Guidance for Industry).

It has been known that nitroso compounds may be present as impurities in angiotensin II receptor blockers (ARBs), ranitidine, nizatidine, and metformin. The inventors examined the possibility of nitroso compounds being formed in other known active pharmaceutical ingredients besides these and found that many active pharmaceutical ingredients have the potential to generate nitroso compounds during the formulation process.

Based on the process by which nitroso compounds are generated, active pharmaceutical ingredients can be classified into the following 10 categories.

1. Compounds known to generate nitroso compounds such as metformin hydrochloride
2. Compounds such as ranitidine that contain NO and amines in the structure of the active pharmaceutical ingredient and may generate known nitroso compounds
3. Compounds such as sultanates containing a tetrazole ring in the structure of the active pharmaceutical ingredient
4. Compounds that may contain residual solvents such as dimethylamine and triethylamine that contribute to the generation of known nitroso compounds due to a synthetic route of the active pharmaceutical ingredient
5. Compounds containing amine structures such as dimethylamine skeleton that generate known nitroso compounds
6. Compounds like ranitidine that contain both amine source and NO source as reaction initiators in their structure, and the active pharmaceutical ingredient itself may be nitrosated
7. Compounds that contain secondary amines in the structure of the active pharmaceutical ingredient and the active pharmaceutical ingredient itself may be nitrosated
8. Compounds that contain tertiary amines in the structure of the active pharmaceutical ingredient and the active pharmaceutical ingredient itself may be nitrosated
9. Compounds with a skeleton other than the amine skeleton, compounds in which NOx is used in the synthetic route, and compounds that may generate unknown nitroso compounds such as EDTA
10. Compounds that do not contain amine sources and NO sources

As a result of the inventors' study, it is clear that many active pharmaceutical ingredients fall into the above categories 1 to 9 and have the potential to generate nitroso compounds. Therefore, the inventors found that in order to reduce nitroso compounds in pharmaceutical compositions or formulations, it is extremely important to control the amount of NOx sources, that is, reactive NOx, that contribute to the generation of nitroso compounds in additive agents used in combination with the active pharmaceutical ingredient.

In addition, a comprehensive evaluation of the amount of nitrite in additive agents in Non Patent Literature 1 reported that nitrite was detected in many additive agents. From this, it is clear that it is extremely important to control the amount of reactive NOx in additive agents in order to reduce nitroso compounds in pharmaceutical compositions or formulations.

[Method for quantifying reactive NOx]

In this embodiment, one selected from an active pharmaceutical ingredient, a pharmaceutically acceptable additive, a pharmaceutical composition, and a formulation can be used as the sample. In addition, in the following embodiments, although an example in which one selected from an active pharmaceutical ingredient, a pharmaceutically acceptable additive, a pharmaceutical composition, and a formulation is used as a sample is shown and described, the present invention is not limited thereto and can also be used as a method for quantifying reactive NOx for food and food additives.

FIG. 1 is a flow diagram of the method for quantifying reactive NOx according to this embodiment. In the method for quantifying reactive NOx according to this embodiment, a sample is contacted with an amine source to produce a nitroso compound (Step S11). In one embodiment, 500 mg of the sample and 5 μl of a 50% solution of an amine source are added to a sealable container and mixed to bring the sample and amine source into contact.

Secondary, tertiary, or quaternary amines may be used as the amine source. In addition, in one embodiment, an active pharmaceutical ingredient or a pharmaceutically acceptable additive having an amine structure to be added to the pharmaceutical composition or formulation may also be used as an amine source from the viewpoint of suppressing the generation of nitroso compounds in the pharmaceutical composition or formulation.

The sample in contact with the amine source is heated under a sealed condition to generate nitroso compounds (Step S12). The term "sealed condition" indicates a state in which the sample is sealed in a hermetically sealed container or a state in which the container containing the sample is tightly closed.

In this embodiment, the sample should be heated to 60°C for the purpose of promoting nitrosation of the amine source by reactive NOx in the sample. In one embodiment, a thermostatic chamber at 60°C and 60% relative humidity (60% RH) is used to store the sample in contact with the amine source under a sealed condition. A storage period is, for example, 2 days, 3 days, 1 week, 2 weeks or 4 weeks.

An appropriate solvent is added to the sample after a specified period of storage to extract nitroso compounds. Solid components such as the sample are removed from the extracted solution using a solid-liquid separation means such as a filter to obtain a sample solution (Step S13). The solvent can be appropriately selected from solvents that can dissolve the nitroso compound and do not affect the quantitation of the nitroso compound. In one embodiment, a water/methanol mixture (7:3) can be used.

The nitroso compounds are quantified in the sample solution (Step S14). In one embodiment, a liquid chromatography tandem mass spectrometer (LC-MS/MS) may be used to quantify the nitroso compounds. In addition, in this embodiment, the method for quantifying nitroso compounds is not limited to the LC-MS/MS.

In this embodiment, the amount of nitroso compounds is converted with the molecular weight of nitrite ions (Step S15). The converted value is used as the amount of reactive NOx.

The amount of reactive NOx in the sample obtained can be stored in a database (Step S16).

In this embodiment, only reactive NOx sources that contribute to the generation of nitroso compounds are quantified, rather than identifying each NOx source contained in the sample one by one. In other words, less reactive NOx sources that do not contribute to the generation of nitroso compounds are not quantified. Therefore, it is possible to predict the amount of nitroso compounds that will be generated in the case where the pharmaceutical composition or formulation is actually manufactured, and the method for quantifying reactive NOx according to this embodiment is useful for the examination of formulas that suppress the generation of nitroso compounds.

According to the method for quantifying reactive NOx according to this embodiment, it is not only possible to measure the amount of reactive NOx in a sample, but also to confirm the possibility of nitrosation of the sample. Even if the sample itself is a compound with a structure that does not nitrosate, the possibility that a nitroso compound is generated by impurities, and the like contained in the sample can be confirmed. As described above, the method for quantifying reactive NOx in this embodiment is an extremely practical and simple quantitation method that does not require specifying the type of NOx source (nitrous acid, NOx other than nitrous acid, or the like) contained in the sample, nor does it require considering the reactivity of each NOx source in the sample.

[Method for Considering Formulas with Reduced Nitroso Compounds]

Using the amount of reactive NOx in the sample quantified by the method for quantifying reactive NOx described above, it is possible to consider the formula in which the amount of nitroso compounds in the pharmaceutical composition or formulation is below the acceptable limit value. In one embodiment, the amount of reactive NOx in the measured sample can be used as a reference to determine the formula of the pharmaceutical composition or formulation.

In more detail, in this embodiment, it is possible to control the amount of nitroso compounds in the pharmaceutical composition or formulation below the acceptable limit value by selecting and combining additive agents that contain a low amount of reactive NOx. In one embodiment, it is preferred to select a combination of additive agents such that the amount of reactive NOx is below 50 ng/tab.

In addition, in one embodiment, antioxidants are known to suppress the generation of nitroso compounds and can be used in combination with additive agents with calculated reactive NOx.

Conventionally, it has been difficult to control the amount of nitroso compounds generated in tablets. By using the method for considering formulas according to this embodiment, the amount of nitroso compounds generated in pharmaceutical compositions or formulations can be kept below the acceptable limit value.

[Formulations Suppressing Generation of Nitroso Compounds]

By using the method for considering formulas according to the embodiments described above, a pharmaceutical composition or formulation in which the generation of nitroso compounds is suppressed can be obtained. In this specification, the term "pharmaceutical composition" is a mixture of an active pharmaceutical ingredient and one or more pharmaceutically acceptable additive agents. In addition, in this specification, the term "formulation" indicates a dosage form such as granules or dry syrup made by granulating the pharmaceutical composition, an injection form made by dissolving the pharmaceutical composition in a pharmaceutically acceptable solvent, a tablet prepared by compression molding the pharmaceutical composition, or a capsule made by sealing the pharmaceutical composition in a capsule. In addition, powders in which the pharmaceutical composition is used as it is are also included in the formulation.

This formulation includes a pharmaceutical composition containing an active pharmaceutical ingredient or a pharmaceutically acceptable additive that has been evaluated by the reactive NOx quantitation method.

In addition, in one embodiment, it is also preferable that the solvent used in the manufacture of the pharmaceutical composition or formulation is also selected from the solvents evaluated by the method for quantifying reactive NOx described above, and it is more preferable that the solvent is selected from the solvents with the lower amount of reactive NOx.

In addition, in the case where wet granulation or spray drying methods are used, nitrosation reactions may be accelerated. In one embodiment, in the case where an additive agent containing more reactive NOx is selected to obtain desired formulation characteristics, the formulation can also be manufactured by a direct tableting method, in which the active pharmaceutical ingredient and the pharmaceutically acceptable additive are mixed and compression molded, or by a dry granulation method, for the purpose of suppressing a nitrosation reaction.

In this embodiment, by using the total amount of substance of reactive NOx (mol/Tab) rather than the total amount of reactive NOx per tablet (μg/Tab), a relationship with the acceptable limit value of nitroso compounds (mol/Tab) generated in the formulation can be clearly confirmed. In other words, since the theoretical value of the substance amount of nitroso compounds can be derived by assuming that all of the reactive NOx contained in one tablet is used for nitrosation of the amine source, the theoretical value (mol/Tab) and the acceptable limit value (mol/Tab) can be compared, and the amount and type of additive agents can be determined so that the theoretical value (mol/Tab) is below the acceptable limit value (mol/Tab) to manufacture the formulations.

[Example]

[Example 1]

Reactive NOx in known additive agents was quantified using the method for quantifying reactive NOx described above. 500 mg of each additive agent and 5 μl of a 50% solution of dimethylamine as an amine source were added to a vial, tightly sealed, and stored in a thermostatic chamber at 60°C and 60% RH for 3 days, 1 week, 2 weeks or 4 weeks. 10 ml of a water/methanol mixture (7:3) was added to the resulting sample, and stirred for 20 minutes to extract the generated nitroso compounds. The extract was filtered through a 0.45 μm hydrophilic poly(tetrafluoroethylene) membrane to obtain a sample solution.

The sample solution was measured by the LC-MS/MS to quantify NDMA in the sample solution. In addition, Nexera (registered trademark) X2 from Shimadzu Corporation was used as an LC (high performance liquid chromatography), and QTRAP (registered trademark) 5500 from AB SCIEX Corporation was used as the MS/MS (tandem mass spectrometer). The quantified NDMA was converted with the molecular weight of nitrite ions, and the amount of reactive NOx (μg/g) was calculated under the following conditions.

NDMA (molecular weight Mw = 74 g/mol)

Nitrite ion (Mw = 46 g/mol)

Amount of reactive NOx in sample (μg/g) = NDMA (μg/g) x Mw (nitrite ion) / Mw (NDMA)

The amount of reactive NOx (μg/g) in each additive agent measured is shown in Table 1. In addition, Table 1 shows the maximum amount of reactive NOx over the storage period as the amount of reactive NOx.

[Table 1]

| Additive agents | Amount of reactive NOx (μg/g) |
|---|---|
| Lactose hydrate | 1.4 |
| Mannitol | 0.021 |
| Microcrystalline cellulose | 1.7 |
| Anhydrous dibasic calcium phosphate | 0.31 |
| Hydroxypropyl cellulose | 1.3 |
| Povidone | 0.76 |
| Corn starch | 0.69 |
| Calcium carmellose | 0.33 |
| Partly pregelatinized starch | 1.6 |
| Sodium starch glycolate | 0.15 |
| Crospovidone | 1 |
| Croscarmellose sodium | 0.076 |
| Hydrated silicon dioxide | 0.28 |
| Magnesium stearate | 0.1 |
| Sodium stearyl fumarate | 0.083 |
| Polyvinyl alcohol | 0.14 |
| Hypromellose | 1.3 |
| Talc | 14 |
| Red ferric oxide | 1.2 |
| Yellow ferric oxide A | 18 |

Table 2 shows the amount of reactive NOx (μg/g) in each additive agent at each storage period.

[Table 2]

| Additive agents | Amount of reactive NOx (μg/g) | | | | |
|---|---|---|---|---|---|
| | 60°C 3 days | 60°C 1 week | 60°C 2 weeks | 60°C 4 weeks | Maximum amount |
| Lactose hydrate | 0.65 | 0.85 | 1.38 | 1.42 | 1.4 |
| Anhydrous dibasic calcium phosphate | N/A | 0.06 | 0.10 | 0.31 | 0.31 |
| Low-substituted hydroxypropyl cellulose | 0.42 | 0.76 | 1.11 | 0.87 | 1.1 |
| Yellow ferric oxide A | 0.27 | 14.96 | 17.66 | 17.45 | 17.7 |
| Yellow ferric oxide B | 0.39 | 0.66 | 0.69 | 0.52 | 0.69 |
| Magnesium stearate | 0.01 | 0.06 | 0.09 | 0.10 | 0.10 |

[Comparative Example 1]

The formulation of formula A shown in FIG. 2 was manufactured by the direct tableting method using an active

pharmaceutical ingredient 1 and an additive agent in which reactive NOx was quantified.

[Example 2]

The formulation of formula B shown in FIG. 2 was manufactured by the direct tableting method using an active pharmaceutical ingredient 1 and an additive agent in which reactive NOx was quantified.

[Comparative Example 2]

The formulation of formula A shown in FIG. 2 was manufactured by the direct tableting method using an active pharmaceutical ingredient 2, which is different from the active pharmaceutical ingredient 1, and an additive agent in which reactive NOx was quantified.

[Example 3]

The formulation of formula B shown in FIG. 2 was manufactured by the direct tableting method using the active pharmaceutical ingredient 2 and an additive agent in which quantified reactive NOx was quantified.

The formulations of Comparative examples 1 to 2 and Examples 2 to 3 were packaged in PTP sheets and aluminum bags and stored under 25°C, 60% RH or 40°C, 75% RH conditions for 26 weeks. Nitrosamines in the formulations after storage were measured by the method described above to quantify the nitrosamines (ng/tab) in the formulations.

FIG. 3 shows the transition of the amount of nitroso compounds when the formulations of the Comparative example 1 and the Example 2 were stored at 25°C and 60% RH. FIG. 4 shows the transition of the amount of nitroso compounds when the formulations of Comparative example 1 and Example 2 were stored at 40°C and 75% RH.

FIG. 5 shows the transition of the amount of nitroso compounds when the formulations of the Comparative example 2 and the Example 3 were stored at 25°C and 60% RH. FIG. 6 shows the transition of the amount of nitroso compounds when the formulations of the Comparative example 2 and the Example 3 were stored at 40°C and 75% RH.

A value converting an acceptable limit value of 96 ng/day of nitroso compounds in the active pharmaceutical ingredient 1 into a value per tablet is 10.7 ng/Tab. The amount of nitroso compounds generated when all reactive NOx in the formulations of the Comparative example 1 and the Example 2 react is 370 ng/Tab and 80 ng/Tab, respectively. Even though these values are higher than the acceptable limit for nitroso compounds (10.7 ng/Tab), as shown in the results of FIG. 3 and FIG. 4, in the formulation of the Example 2, the generation of nitroso compounds was significantly suppressed and was below the acceptable limit value.

In addition, the amount of nitroso compounds generated when all reactive NOx in the formulations of the Comparative example 2 and the Example 3 react is 1700 ng/Tab and 370 ng/Tab, respectively, while the acceptable limit value of nitroso compounds in the active pharmaceutical ingredient 2 is 400 ng/Tab. As shown in the results of FIG. 5 and FIG. 6, in the formulation of the Example 3, the generation of nitroso compounds was significantly suppressed.

## Claims

**1.** A method for quantifying reactive NOx comprising:
contacting a sample with an amine source to generate a nitroso compound.

**2.** The method for quantifying reactive NOx according to claim 1, wherein the sample contacted with the amine source under a sealed condition is heated to generate the nitroso compound.

**3.** The method for quantifying reactive NOx according to claim 1, wherein the nitroso compound is quantified, and converted by a molecular weight of nitrite ion.

**4.** The method for quantifying reactive NOx according to claim 1, wherein the amine source is a secondary amine, a tertiary amine, or a quaternary amine.

**5.** The method for quantifying reactive NOx according to any one of claims 1 to 4, wherein the sample is one selected from a group consisting of an active pharmaceutical ingredient, a pharmaceutically acceptable additive agent, a pharmaceutical composition, and a formulation.

**6.** A formulation comprising: an active pharmaceutical ingredient or a pharmaceutically acceptable additive that has

been evaluated by the method for quantifying reactive NOx according to claim 5.

7. The formulation according to claim 6, wherein the formulation contains the active pharmaceutical ingredient or the pharmaceutically acceptable additive agent in which a theoretical value of a nitroso compound generated in the formulation is calculated as an amount of substance per tablet by using a total amount of reactive NOx per tablet and the theoretical value is below an allowable limit value of the nitroso compound calculated as the amount of substance per tablet.

FIG. 1

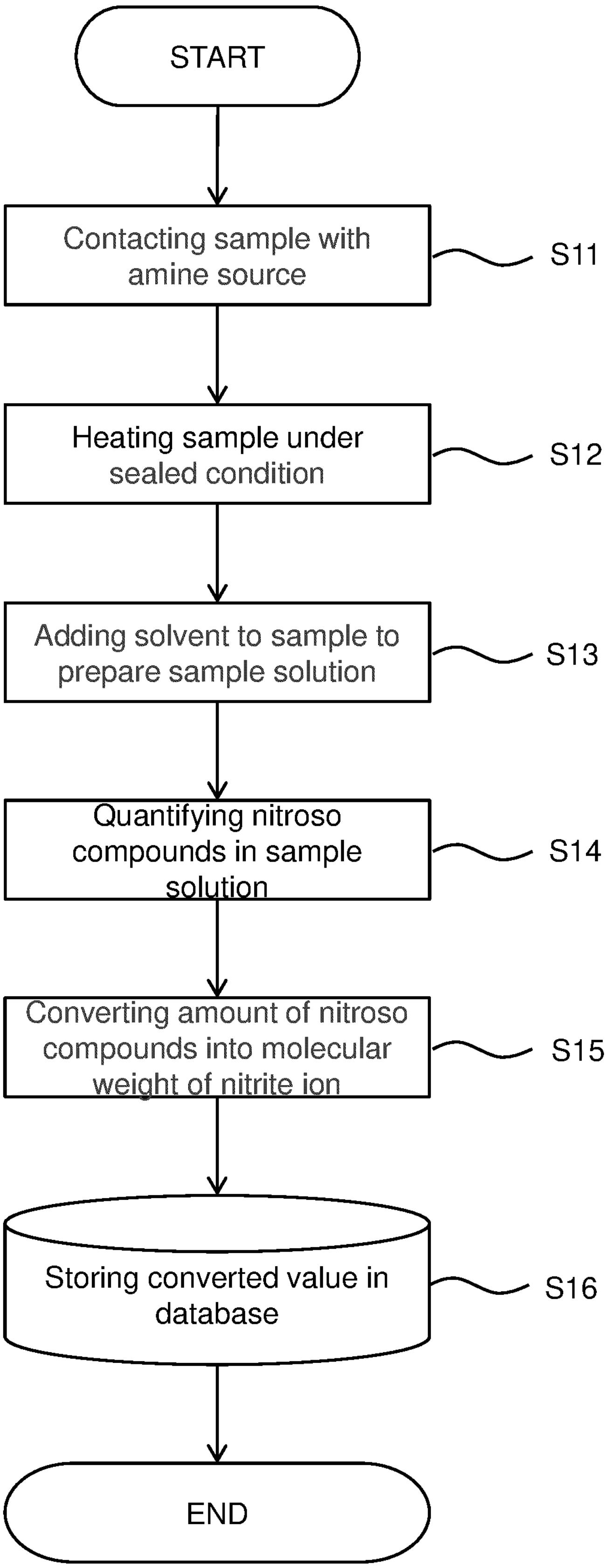
START
Contacting sample with amine source
S11
Heating sample under sealed condition
S12
Adding solvent to sample to prepare sample solution
S13
Quantifying nitroso compounds in sample solution
S14
Converting amount of nitroso compounds into molecular weight of nitrite ion
S15
Storing converted value in database
S16
END

FIG. 2

| Name | Reactive NOx (ppm) | Active pharmaceutical ingredient 1 | | | | Active pharmaceutical ingredient 2 | | | |
|---|---|---|---|---|---|---|---|---|---|
| | | Formulation A | | Formulation B | | Formulation A | | Formulation B | |
| | | Content (mg/tab) | Reactive NOx content (ng) | Content (mg/tab) | Reactive NOx content (ng) | Content (mg/tab) | Reactive NOx content (ng) | Content (mg/tab) | Reactive NOx content (ng) |
| Active pharmaceutical ingredient 1 | — | 250 | — | 250 | — | 0 | — | 0 | — |
| Active pharmaceutical ingredient 2 | — | 0 | — | 0 | — | 5 | — | 5 | — |
| Lactose hydrate | 1.4 | 88 | 123.2 | 0 | — | 88 | 123.2 | 0 | — |
| Anhydrous dibasic calcium phosphate | 0.31 | 0 | — | 88 | 27.28 | 0 | — | 88 | 27.28 |
| Low-substituted hydroxypropyl cellulose | 1.1 | 15 | 16.5 | 15 | 16.5 | 15 | 16.5 | 15 | 16.5 |
| Yellow ferric oxide A | 17.7 | 5 | 88.5 | 0 | — | 5 | 88.5 | 0 | — |
| Yellow ferric oxide B | 0.69 | 0 | — | 5 | 3.45 | 0 | — | 5 | 3.45 |
| St-Mg | 0.1 | 4 | 0.4 | 4 | 0.4 | 2 | 0.2 | 2 | 0.2 |
| Total reactive NOx | | | 229 | | 48 | | 228 | | 47 |

FIG. 3

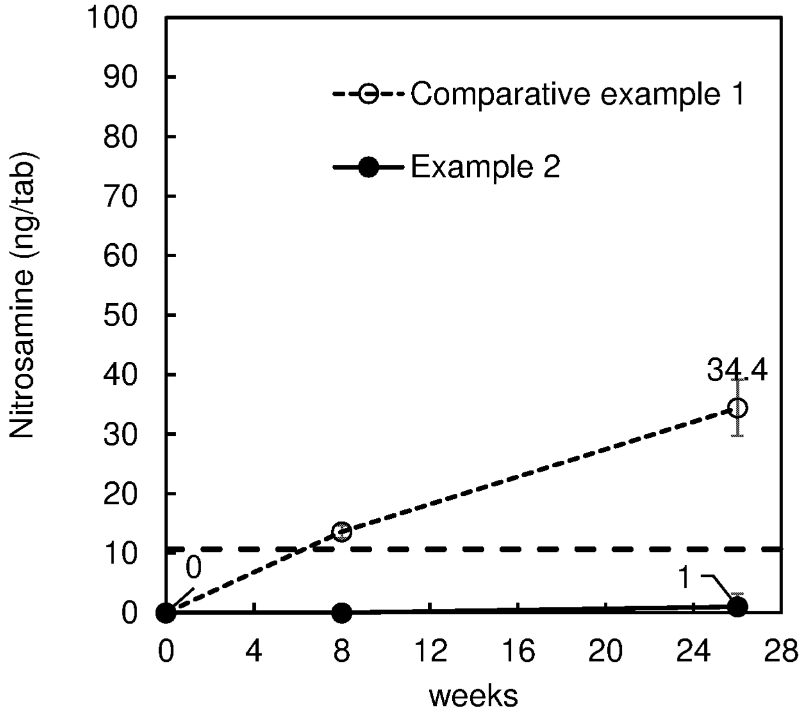
100
90
80
70
60
50
40
30
20
10
0
Nitrosamine (ng/tab)
Comparative example 1
Example 2
34.4
0
1
0
4
8
12
16
20
24
28
weeks

FIG. 4

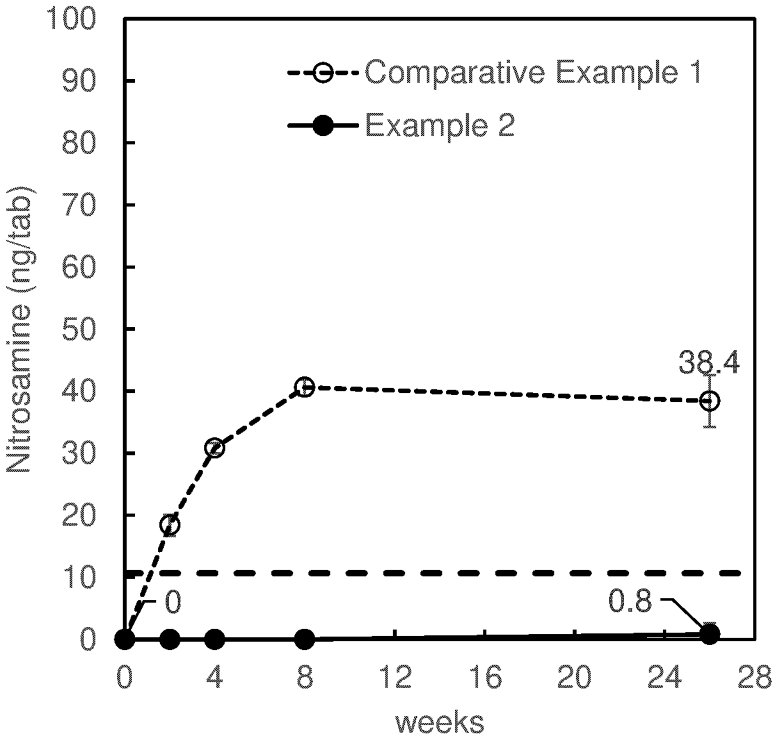
100
90
80
70
60
50
40
30
20
10
0
Nitrosamine (ng/tab)
Comparative Example 1
Example 2
38.4
0
0.8
0
4
8
12
16
20
24
28
weeks

FIG. 5

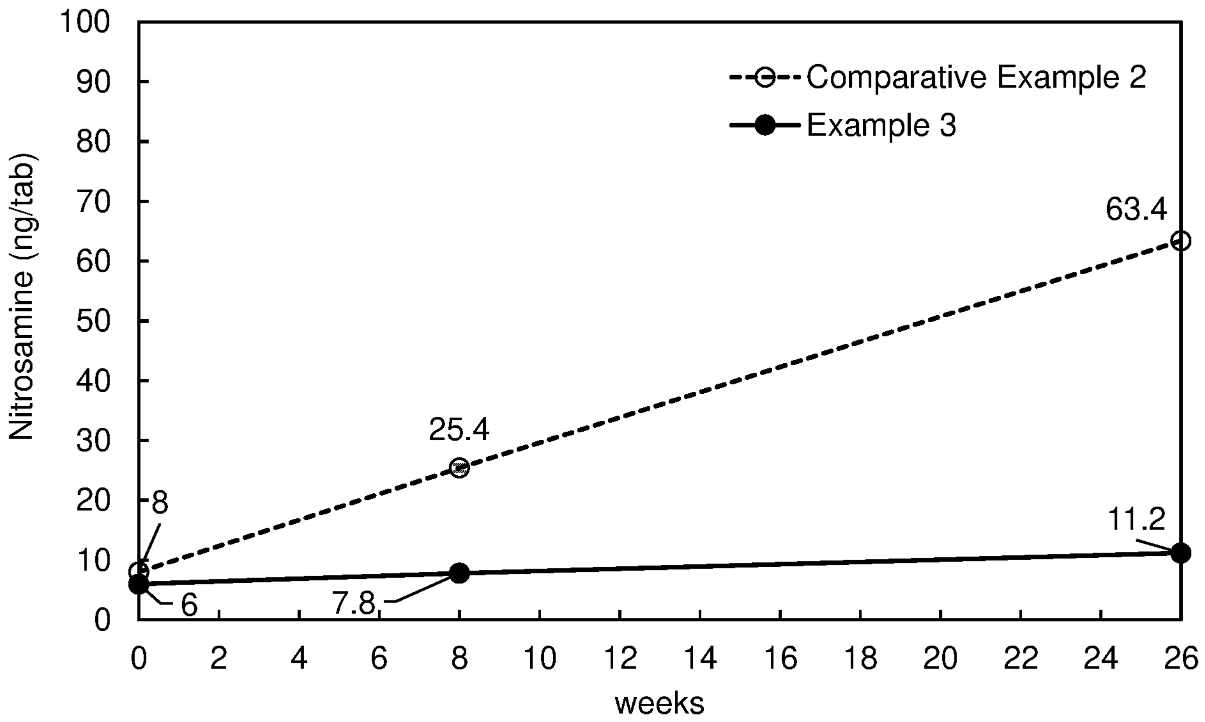
100
90
80
70
60
50
40
30
20
10
0
Nitrosamine (ng/tab)
Comparative Example 2
Example 3
63.4
25.4
8
11.2
6
7.8
0
2
4
6
8
10
12
14
16
18
20
22
24
26
weeks

FIG. 6

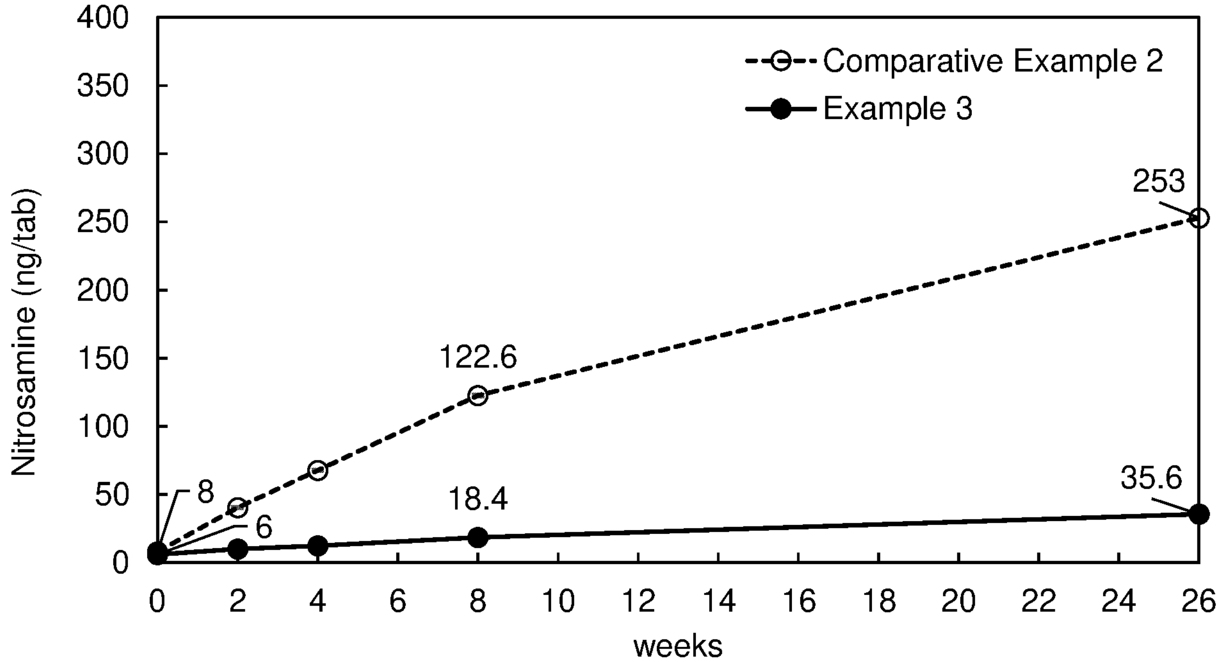
Comparative Example 2
Example 3
Nitrosamine (ng/tab)
400
350
300
250
200
150
100
50
0
0 2 4 6 8 10 12 14 16 18 20 22 24 26
weeks
8
6
122.6
18.4
253
35.6

**INTERNATIONAL SEARCH REPORT**

International application No.
**PCT/JP2023/036245**

**A. CLASSIFICATION OF SUBJECT MATTER**

***G01N 31/00***(2006.01)i; ***A61K 47/16***(2006.01)i; ***G01N 33/15***(2006.01)i
FI: G01N31/00 J; G01N33/15 Z; A61K47/16

According to International Patent Classification (IPC) or to both national classification and IPC

**B. FIELDS SEARCHED**

Minimum documentation searched (classification system followed by classification symbols)

G01N31/00; A61K47/16; G01N33/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | 西田宏, α－ナフチルアミンによる亜硝酸の検出, 分析化学, 05 April 1964, vol. 13, no. 4, pp. 361-362<br>2. Analysis operation, (BUNSEKI KAGAKU), non-official translation (NISHIDA, Hiroshi. Detection of nitrite by α-Naphthylamine) | 1-4 |
| Y | | 5 |
| Y | 亜硝酸塩及び亜硝酸イオン測定の重要性, Thermo Fisher Scientific [online], 06 January 2022 [retrieval date 05 December 2023], internet:<URL: https://www.thermofisher.com/blog/learning-at-the-bench/nitrite_ic_cmd_mkt/><br>・Measurement of nitrite and nitrite ion, ・Method for measuring nitrite ions, non-official translation (Importance of measuring nitrite and nitrite ions) | 5 |
| X | | 6-7 |
| Y | ニトロソアミンのサポートについて, DFE Pharma [online], 24 August 2022 [retrieval date 05 December 2023], internet:<URL: https://dfepharma.com/jp/news/about-nitrosamines/><br>Your Support on Nitrosamines, (Your Support on Nitrosamines) | 5 |
| X | | 6-7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 December 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/036245**

**C. DOCUMENTS CONSIDERED TO BE RELEVANT**

| Category* | Citation of document, with indication, where appropriate, of the relevant passages | Relevant to claim No. |
|---|---|---|
| Y<br><br><br><br><br>X | JIRES, Jakub. Nitrites as precursors of N-nitrosation in pharmaceutical samples - A trace level analysis. Journal of Pharmaceutical and Biomedical Analysis. 18 February 2022, vol. 213, p. 114677<br>abstract, fig. 1, 3.2.6. The UPLC-MS determination of nitrite content in pharmaceutical samples, 4. Conclusion | 5<br><br><br><br><br>6-7 |
| A | JP 11-118783 A (NICHIRO CORP) 30 April 1999 (1999-04-30)<br>abstract | 1-7 |
| A | JP 62-294943 A (LION CORP) 22 December 1987 (1987-12-22)<br>examples | 1-7 |
| A | JP 2014-508108 A (BASF SE) 03 April 2014 (2014-04-03)<br>abstract | 1-7 |
| A | NANDA, Kausik K. Inhibition of N-Nitrosamine Formation in Drug Products: A Model study. Journal of Pharmaceutical Sciences. 14 August 2021, vol. 110, pp. 3773-3775<br>entire text | 1-7 |
| P, A | 医薬品におけるニトロソアミン類生成のリスク軽減について: 医薬品添加剤の役割, メグレ・ジャパン株式会社 [オンライン], 15 August 2023 [retrieval date 05 December 2023], internet:<URL: https://meggle.co.jp/wordpress/wp-content/uploads/2023/08/%E5%8C%BB%E8%96%AC%E5%93%81%E3%81%AB%E3%81%8A%E3%81%91%E3%82%8B%E3%83%8B%E3%83%88%E3%83%AD%E3%82%BD%E3%82%A2%E3%83%9F%E3%83%B3%E9%A1%9E%E7%94%9F%E6%88%90%E3%81%AE-%E3%83%AA%E3%82%B9%E3%82%AF%E8%BB%BD%E6%B8%9B%E3%81%AB%E3%81%A4%E3%81%84%E3%81%A6_%E5%8C%BB%E8%96%AC%E5%93%81%E6%B7%BB%E5%8A%A0%E5%89%A4%E3%81%AE%E5%BD%B9%E5%89%B2-1.pdf><br>entire text, (MEGGLE JAPAN CO., LTD. [online]), non-official translation (Reducing the risk of nitrosoamine formation in pharmaceuticals: the role of pharmaceutical additives) | 1-7 |

Form PCT/ISA/210 (second sheet) (January 2015)

INTERNATIONAL SEARCH REPORT

International application No.
PCT/JP2023/036245

**Box No. III Observations where unity of invention is lacking (Continuation of item 3 of first sheet)**

This International Searching Authority found multiple inventions in this international application, as follows:

While the invention in claim 1 is a method for quantifying reactive NOx, which includes bringing a sample into contact with an amine source to generate a nitroso compound, the invention in claims 6-7 is a preparation containing an additive, and thus the invention in claim 1 does not share the common special technical feature with the invention classified as inventions 6-7.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/036245**

| Patent document cited in search report | Publication date (day/month/year) | Patent family member(s) | Publication date (day/month/year) |
|---|---|---|---|
| JP 11-118783 A | 30 April 1999 | (Family: none) | |
| JP 62-294943 A | 22 December 1987 | (Family: none) | |
| JP 2014-508108 A | 03 April 2014 | WO 2012/076449 A1<br>abstract<br>EP 2649041 A1<br>CN 103313962 A<br>KR 10-2013-0136502 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **R. BOETZEL et al.** A Nitrite Excipient Database: A Useful Tool to Support N-Nitrosamine Risk Assessments for Drug Products. *J. Pharm. Sci*, 2023, vol. 112, 1615-1624